# EUROPEAN PATENT APPLICATION

(11) **EP 4 365 162 A2**
(43) Date of publication of application: **08.05.2024**
(21) Application number: 24159713.7
(22) Date of filing: 20.12.2021
(51) Int. Cl.: C07C 209/00

(54) **A PROCESS FOR THE PREPARATION OF N-(BIS(4-METHOXYPHENYL)METHYL)-6-OXO-2-(PYRIDAZIN-3-YL)-1,6-DIHYDROPYRIMIDINE-5-CARBOXAMIDE**

(30) Priority: 21.12.2020 EP 20215933
(62) Divisional of application: 21840013.3
(71) Applicant: Intervet International B.V., 5831 AN Boxmeer (NL)
(72) Inventor: CHASSAING, Christophe, Pierre, Alain, 55270 Schwabenheim (DE); SALANTA, Daniel, 55270 Schwabenheim (DE)
(74) Representative: Intervet International B.V.

(57) **Abstract**

A process for the preparation of *N*-(bis(4-methoxyphenyl)methyl)-6-oxo-2-(pyridazin-3-yl)-1,6-dihydropyrimidine-5-carboxamide with improved reaction conditions and reduced use of toxic reagents and solvents.

## Description

### Background

The compound of Formula I 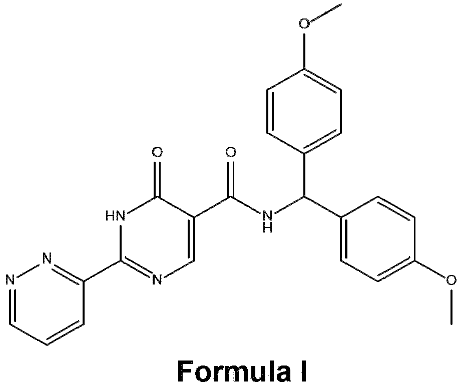 is known for the treatment of anemia. A multistep synthesis to prepare this compound is disclosed in J. Med. Chem. 2016, 59, 11039-11049 by Debenham et al, "Discovery of MK-8617, an Orally Active Pan-Inhibitor of Hypoxia-Inducible Factor Prolyl Hydroxylase 1-3 (HIF PHD1-3) for the Treatment of Anemia".

This compound is named N-(bis(4-methoxyphenyl)methyl)-6-oxo-2-(pyridazin-3-yl)-1,6-dihydropyrimidine-5-carboxamide via ChemDraw version 15.0. It is also called N-[Bis(4-methoxyphenyl)methyl]-4-hydroxy-2-(pyridazin-3-yl)pyrimidine-5-carboxamide.

WO2009117269 (Substituted 4-Hydroxypyrimidine-5-carboxamides) also discloses the structure of Formula I and a method to prepare this compound.

These processes are characterized by reaction times greater than of 24 hours and excess use of toxic reagents and solvents.

### Summary of the Invention

A process of making the compound of Formula I 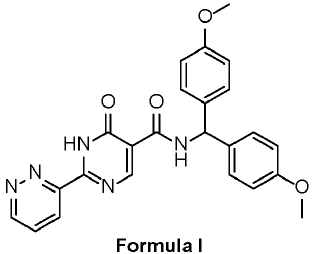 comprising
a) reacting a compound of Formula BMPN 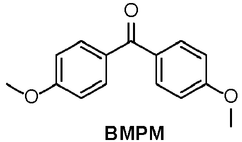 with formamide and formic acid to produce a compound of Formula BMPF 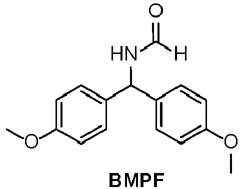
b) hydrolyzing the compound of Formula BMPF with acid to yield the compound of Formula BMMA 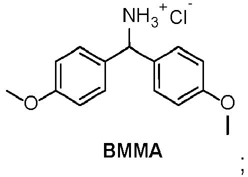 and
c) reacting the compound of Formula BMMA with a compound of Formula DHP 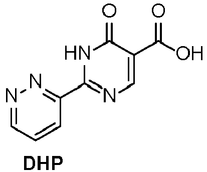 to yield the compound of Formula I.

### Detailed Description

A new process to produce the compound of Formula I has been developed characterized by shorter reaction times and reduced use of solvents and toxic reagents.

The overall reaction scheme is displayed below as Scheme 1. 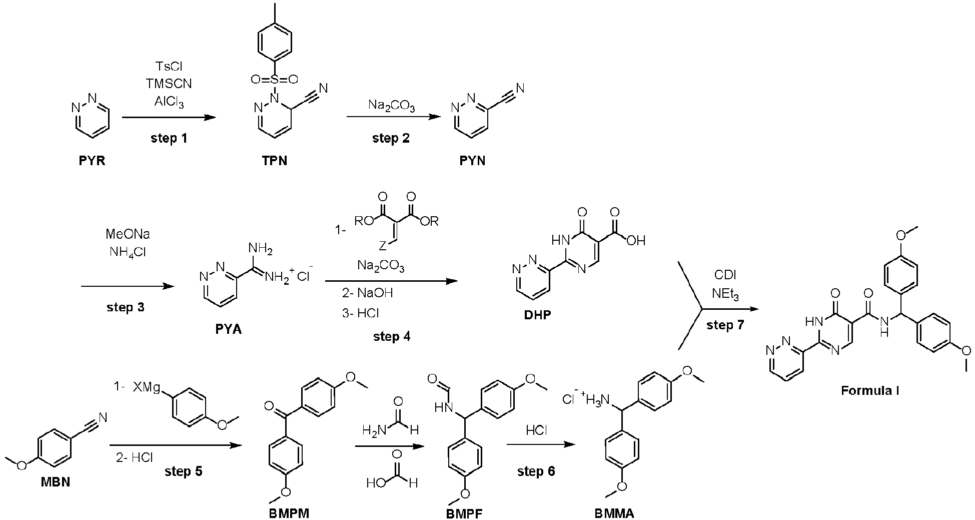
R is C₁-C₄ alkyl
Z is C₁-C₂ alkoxy or R₂NH
X is Br or Cl

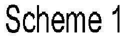

In step 1 of Scheme 1, the process has been improved by reducing the use of trimethylsilyl cyanide, toluenesulfonyl chloride and solvents. In step 3, the process has been improved by reduced reaction time and reduced temperature. During step 4 of Scheme 1, the process has been improved by substituting the use of sodium ethoxide as a base with sodium carbonate as a base which allows a reduction of the reaction time from about 28 h to about 10 h. Finally, a novel sequence of transformations has been developed to produce the intermediate compound of Formula BMMA in steps 5 and 6. The DHP intermediate and the BMMA intermediate are reacted in step 7 to produce the compound of Formula I in improved purity.

An alternative process for the synthesis of the BMMA free base has been developed as shown in Scheme 2

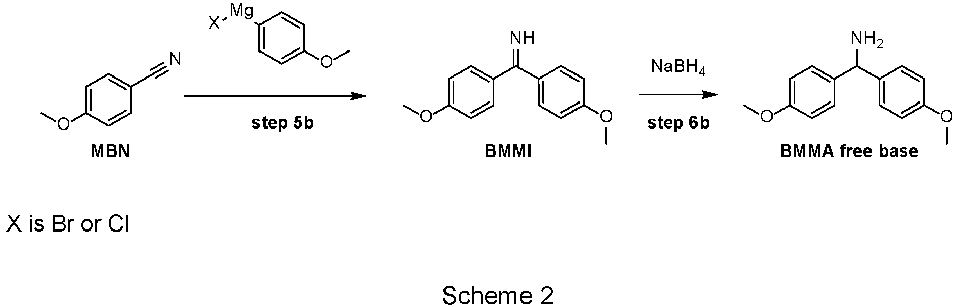

Another embodiment of the invention is a process of making the compound of Formula I 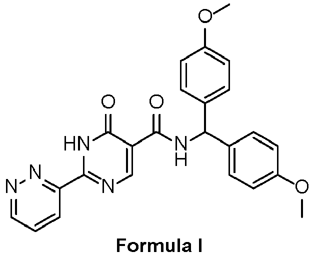 comprising
a) reducing a compound of Formula BMMI 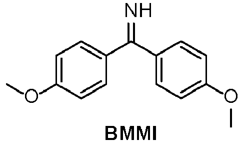 with a reducing agent to yield a compound of Formula BMMA free base 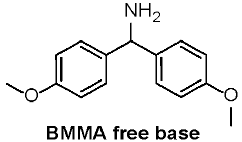
b) reacting the compound of Formula BMMA free base with a compound of Formula DHP 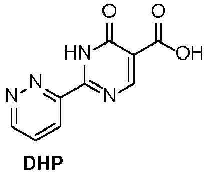 to yield the compound of Formula I.

Another embodiment of the invention is a process of making the compound of Formula I 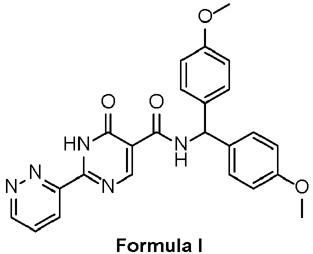 comprising
a) reacting pyridazine with between about 1.0 equivalent and about 1.5 equivalent of each of trimethylsilyl cyanide and toluenesulfonyl chloride to yield a compound of a Formula TPN 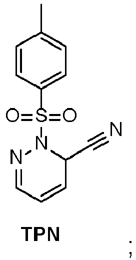
b) washing the compound of Formula TPN with sodium bicarbonate to yield a compound of Formula PYN 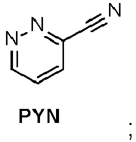
c) reacting the compound of Formula PYN with sodium in methanol at ambient temperature and then adding ammonium chloride to yield a compound of Formula PYA 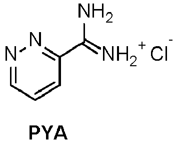
d) reacting the compound of Formula PYA with diethyl ethoxymethylenemalonate and sodium carbonate to yield a compound of Formula DHP 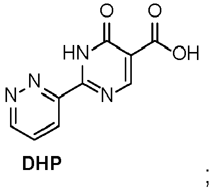 and
e) reacting the compound of Formula DHP with a compound of Formula BMMA 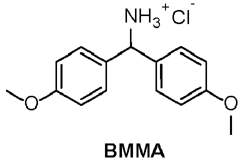 or a free base thereof to yield the compound of Formula I.

In an alternative embodiment, the pH of the process of step e) is reduced to below about 2.0, preferably to 1.0.

In an alternative embodiment, diethyl ethoxymethylenemalonate is replaced with a compound of the below formula 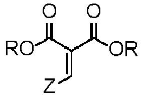 wherein R is C₁-C₄ alkyl and Z is C₁-C₂ alkoxy or R₂NH.

Another embodiment of the invention is a process of making the compound of Formula BMMA 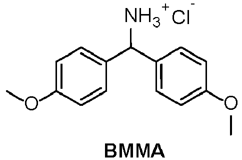 comprising
a) reacting a compound of Formula BMPN 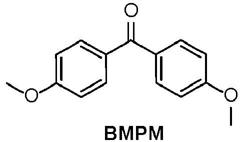 with formamide and formic acid to produce a compound of Formula BMPF 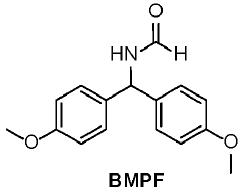 and
b) hydrolyzing the compound of Formula BMPF with acid to yield the salt of the compound of Formula BMMA.

In an alternative embodiment, the acid is selected from sulfuric acid, hydrobromic acid, phosphoric acid or hydrochloric acid.

In an alternative embodiment, step a) of the process is conducted at 130 °C for 48 h or at 155 °C for 10h or 175 °C for 2.5h.

Another embodiment of the invention is a process to make a compound of Formula BMPN 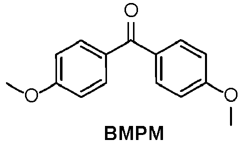 comprising reacting a compound of Formula MBN 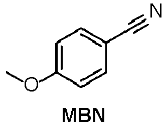 with a compound of the below formula 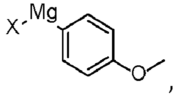 wherein X is Br or Cl, and an acid, preferably hydrochloric acid to yield the compound of Formula MBN.

Another embodiment of the invention is a process of making a compound of Formula BMMA free base 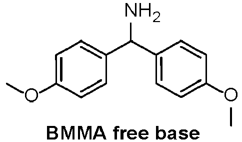 comprising
a) reducing a compound of Formula BMMI 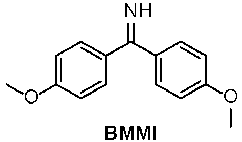 with a reducing agent to yield the compound of Formula BMMA free base.

In an alternative embodiment, the reducing agent is sodium borohydride, a borane complexes, LiAIH4) or catalytic hydrogenation with a Rhodium (Rh) or a Iridium (Ir) catalyst.

Another embodiment of the invention is a process of making a compound of Formula BMMI 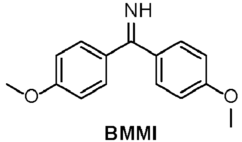 comprising
comprising reacting a compound of Formula MBN 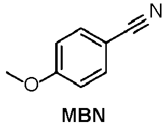
with a compound of the below formula 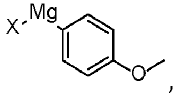 wherein X is Br or Cl,
to yield the compound of Formula BMMI.

Another embodiment of the invention is a process of making a compound of Formula PYN 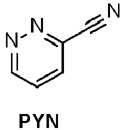 comprising
a) reacting pyridazine with between about 1.0 equivalent and about 1.5 equivalent of each of trimethylsilyl cyanide and toluenesulfonyl chloride to yield a compound of a Formula TPN 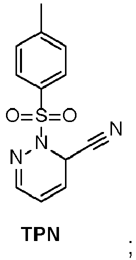 and
b) washing the compound of Formula TPN with sodium bicarbonate to yield the compound of Formula PYN.

In an alternative embodiment, the equivalents of each of trimethylsilyl cyanide and toluenesulfonyl chloride are about 1.2 equivalents.

In an alternative embodiment, the process is conducted in a solvent of between about 8 to 15 volumes.

In an alternative embodiment, the solvent is 2-methyltetrahydrofuran.

In an alternative embodiment, the toluenesulfonyl chloride is replaced with methane sulfonyl chloride.

In an alternative embodiment, the process of step a) further comprises adding aluminum trichloride in two, three or four portions.

Another embodiment of the invention is a process of making a compound of Formula PYA 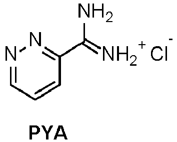 comprising
a) reacting a compound of Formula PYN with sodium in methanol at ambient temperature without reflux; and
b) adding ammonium chloride
to yield the compound of Formula PYA.

Another embodiment of the invention is a process of making a compound of Formula DHP 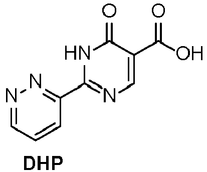 comprising reacting a compound of Formula PYA with diethyl ethoxymethylenemalonate and sodium carbonate to yield the compound of Formula DHP.

In an alternative embodiment, the pH of the process is reduced to below about 3.5, preferably to 1.0.

In an alternative embodiment, diethyl ethoxymethylenemalonate is replaced with a compound of the below formula 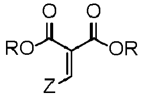 wherein R is C₁-C₄ alkyl and Z is C₁-C₂ alkoxy or R₂NH.

### Advantages of the inventive processes

The synthesis of pyridazine-3-carbonitrile (PYN) developed as part of the inventive process has several advantages over the previously disclosed synthesis for this compound. The amount of trimethylsilylcyanide (TMS-CN) used in the inventive process is significantly lower than that used in the previous processes. The same is true for the amounts of tosyl chloride and aluminum trichloride. It was also discovered that adding the aluminum trichloride in portions allowed a higher conversion to PYN. The solvent for this reaction has been changed from dichloromethane (DCM) to the less toxic 2-methyltetrahydrofuran (Me-THF). Moreover, the volume of solvent used has also been reduced to less than half. These changes have resulted in reduced reaction time for the formation of TPN from greater than 60 hours to less than 20 hours. Furthermore, the product may be obtained by precipitation without additional extraction or chromatography.

The synthesis of amino(pyridazin-3-yl)methaniminium chloride PYA also has several advantages over the previous processes. The reaction time has been lowered to less than 3 hours even though it was conducted at room temperature. In the disclosed previous processes, this reaction either lasted greater than 24 hours at room temperature or required heating to achieve lower reaction times. The volume of solvent has also been reduced.

In the current synthesis of 4-Hydroxy-2-(pyridazin-3-yl)pyrimidine-5-carboxylic acid (DHP), the base used is sodium carbonate. The previous processes used sodium ethoxide. This change reduced the reaction time from over 24 hours to less than 10 hours.

It has been discovered that bis(4-Methoxyphenyl)methanamine hydrochloride (BMMA) can be advantageously prepared from bis(4-methoxyphenyl)methanone (BMPM) via a Leuckart reaction followed by an acidic hydrolysis of the intermediate *N*-(bis(4-methoxyphenyl)methyl)formamide (BMPF); thus providing a novel economic and metal free alternative to the known process. This is in contrast to the previous process where BMMA could be obtained by reacting BMPM with hydroxylamine and by reducing the obtained oxyme to the desired amine in the presence of hydrogen and of a palladium catalyst.

Abbreviations Used in the Description of the Preparation of the Compounds of the Present Invention:

| Abbreviations | Definition |
|---|---|
| AlCl₃ | Aluminum trichloride |
| CDI | 1, 1 '-carbonyldiimidazole |
| DCM | Dichloromethane |
| EtAOc | Ethyl Acetate |
| EtOH | ethanol |
| HCl | Hydrochloric acid |
| LiAlH₄ | Lithium Aluminum Hydride |
| MeOH | Methanol |
| MeONa | Sodium methoxide |
| Me-THF | 2-methyltetrahydrofuran |
| NaOH | Sodium hydroxide |
| NaBH₄ | sodium borohydride |
| Na₂CO₃ | sodium carbonate |
| NH₄Cl | Ammonium chloride |
| NEt₃ | Triethyl amine |
| NMP | *N*-methyl-2-pyrrolidone |
| TMSCN | trimethylsilylcyanide |
| TsCl | tosyl chloride |
| THF | Tetrahydrofuran |
| PYR | pyridazine |
| TPN | 2-[(4-Methylphenyl)sulfonyl]-2,3-dihydropyridazine-3-car-bonitrile |
| PYN | pyridazine-3-carbonitrile |
| PYA | amino(pyridazin-3-yl)methaniminium chloride |
| BMPM | bis(4-methoxyphenyl)methanone |
| BMPF | *N*-(bis(4-methoxyphenyl)methyl)formamide, CAS N° 126748-44-5 |
| BMMA | bis(4-Methoxyphenyl)methanamine hydrochloride |
| BMMI | bis(4-Methoxyphenyl)methanimine, CAS N° 5291-48-5 |
| MBN | 4-methoxybenzonitrile |
| DHP | 4-Hydroxy-2-(pyridazin-3-yl)pyrim idine-5-carboxylic acid |

A reducing agent is a substance that brings about reduction by being oxidized and losing electrons.

Borane complexes are types of reducing agents. Examples are diborane (B₂H₆), borane-tetrahydrofuran (BTHF) and borane-dimethyl sulfide (BMS, DMSB).

Volumes of solvents means the number of liters (L) of solvent required for each kilogram (Kg) of starting material. For example, 0.1 kg of pyridazine in 0.9 L of 2-methyltetrahydrofuran means 9 volumes of solvent.

### Examples

### Example 1 Preparation of pyridazine-3-carbonitrile (PYN)

A reactor was charged with 2-methyltetrahydrofuran (500 mL), pyridazine (90 mL, 1.25 mol), trimethylsilylcyanide (204 mL, 1.52 mol) and aluminum trichloride (1.67 g, 12.49 mmol) and the content was cooled to 0 °C. A solution of tosyl chloride (286 g, 1.50 mol) in 2-methyltetrahydrofuran (400 mL) was added dropwise within 90 min. Stirring was continued for 16 h while the mixture was allowed to reach room temperature. Aluminum trichloride (1.67 g, 12.49 mmol) was added and the reaction mixture was stirred at room temperature for 5 h. The reaction mixture was filtered, and the wet cake was rinsed with ethanol (250 mL). The wet cake was dried under reduced pressure at 40 °C to afford a solid (229 g). The isolated solid was suspended in a mixture of 2-methyltetrahydrofuran (1.15 L) and of aqueous saturated sodium carbonate (915 mL) and the mixture was vigorously stirred. After 4 h reaction time, water (30 mL) was added to facilitate stirring. After additional 2 h reaction time, stirring was stopped and the layers were allowed to settle. The aqueous layer was extracted twice with ethyl acetate (250 mL). The combined organic layers were dried with brine (100 mL) and were concentrated under reduced pressure at 40 °C to afford pyridazine-3-carbonitrile (83 g, 0.79 mol).
HPLC Method A_BEHC18: Ret. Time: 0.43 min; m/z 106 (pos)
¹H NMR (400 MHz, CDCl₃) δ (ppm): 9.40 (1H, J = 1.6, 5.2 Hz, dd); 7.90 (1H, J = 1.6, 7.6 Hz, dd); 7.71 (1H, J = 5.2, 7.6 Hz, dd).

### Example 2 Preparation of amino(pyridazin-3-yl)methaniminium chloride (PYA)

A reactor was charged with methanol (1 L) and sodium (4.37 g, 0.19 mol) was added under stirring at room temperature. After complete dissolution of sodium, pyridazine-3-carbonitrile (200 g, 1.90 mol) was added. An increase in temperature to 45 °C is observed. The reaction mixture was stirred for 30 min at room temperature and ammonium chloride (112 g, 2.09 mol) was added. Stirring was continued for 2 h. The precipitate formed was filtered and the filtrate was partially concentrated under reduced pressure to induce a new precipitation. After filtration of the suspension, the combined solids were rinsed with ethyl acetate (400 mL) and were dried under reduced pressure at 40 °C to afford amino(pyridazin-3-yl)methaniminium chloride (267 g, 1.68 mol).
HPLC Method A_BEHC18: Ret. Time: 0.20 min; m/z 123 (pos)
¹H NMR (400 MHz, CDCl₃) δ (ppm): 9.58 (1H, J = 1.5, 5.0 Hz, dd); 8.60 (1H, J = 1.5, 8.7 Hz, dd); 8.10 (1H, J = 5.0, 8.7 Hz, dd).

### Example 3 Preparation of 4-Hydroxy-2-(pyridazin-3-yl)pyrimidine-5-carboxylic acid (DHP)

A reactor was purged with nitrogen and was charged with ethanol (2,5 L) and amino(pyridazin-3-yl)methaniminium chloride (267 g, 1.68 mol). Sodium carbonate (268 g, 2.53 mol) and diethyl ethoxymethylmalonate (476 mL, 2.36 mol) were added followed by ethanol (2.3 L). The temperature was adjusted to 70 °C and the contents were stirred at this temperature for 2 h. A solution of sodium hydroxide (135 g, 3.37 mol) in water (2.7 L) was added at a speed ensuring good stirability of the mixture. The reaction mixture was stirred at 70 °C for 4.5 h and was then diluted with ethanol (2 L) and water (1 mL) to maintain sufficient stirability of the contents. The reaction mixture was stirred at 70 °C for additional 3 h before the temperature was lowered to 40 °C. The pH of the reaction mixture was adjusted to pH 3.5 by the addition of aqueous 4 N hydrochloric acid (1.5 L). The resulting suspension was filtered and the wet cake was resuspended in pure aqueous 4 N hydrochloric acid (4 L) and was stirred at 70 °C for 3 h. Filtration of the suspension and drying of the wet cake under reduced pressure at 40 °C afforded 4-Hydroxy-2-(pyridazin-3-yl)pyrimidine-5-carboxylic acid (309 g, 1.42 mol).
HPLC Method A_BEHC18: Ret. Time: 0.49 min; m/z 219 (pos)
¹H NMR (400 MHz, CDCl₃) δ (ppm): 9.53 (1H, J = 1.7, 5.0 Hz, dd); 8.59 (1H, s); 8.55 (1H, J = 1.6, 8.6 Hz, dd); 8.04 (1H, J = 5.0, 8.6 Hz, dd).

### Example 4 Preparation of BMMA

### 4A Preparation of bis(4-methoxyphenyl)methanone (BMPM)

A reactor was placed under inert atmosphere (nitrogen) and a 1M solution of methoxyphenyl-magnesiumbromide in tetrahydrofuran (28 L, 28 mol) was charged. 4-Methoxybenzonitrile (2.95 kg, 22.1 mol) was added to the reactor under stirring, the temperature was raised to 60 °C to react and the reaction mixture was stirred at this temperature for 4 h. The temperature was lowered to 15 °C and the reaction mixture was transferred to a second reactor. The first reactor was rinsed with tetrahydrofuran (3 L) which was added to the reaction mixture. A 6N aqueous solution of hydrochloric acid (45 L) was charged into the first reactor and the temperature was adjusted between 0 and 3 °C. The reaction mixture was added to the aqueous hydrochloric acid under stirring while maintaining the temperature of the contents below 45 °C. The second reactor was rinsed with tetrahydrofuran (3 L) which was also added to the reaction mixture. The temperature of the contents of the first reactor was increased to 60 °C and the mixture was stirred at this temperature for 15 h. The heating was stopped and the temperature was allowed to reach 20 °C. The resulting suspension was filtered and the wet cake was rinsed twice with water (5 L) and dried at 40 °C to afford bis(4-methoxyphenyl)methanone (5.18 kg, 21.4 mol).
HPLC Method A_BEHC18: Ret. Time: 1.08 min; m/z 243 (pos)
¹H NMR (400 MHz, CDCl₃) δ (ppm): 7.71 (4H, J = 8.8 Hz, d); 7.09 (4H, J = 8.8 Hz, d); 6.07 (1H, J = 8.7 Hz, d); 3.71 (6H, s).

### 4B Preparation of N-(bis(4-methoxyphenyl)methyl)formamide (BMPF)

A reactor was charged with formamide (2.16 L), formic acid (205 mL, 5.34 mol) and bis(4-methoxyphenyl)methanone (431 g, 1.78 mol). The temperature of the resulting mixture was set to 130 °C and is stirred for 48 h. The temperature was lowered to 120 °C. The reaction mixture was added under vigorous stirring to water (5 L) pre-warmed at 30 °C in a second reactor. The resulting suspension was stirred overnight (about 15 h) and was filtered. The wet cake was rinsed twice with water (1.5 L each) and was dried under reduced pressure at 40 °C to afford *N*-(bis(4-methoxyphenyl)methyl)formamide (470 g, 1.73 mol).
HPLC Method A_BEHC18: Ret. Time: 0.97 min; m/z 227 (pos)
¹H NMR (400 MHz, CDCl₃) δ (ppm): 8.99 (1H, J = 8.7 Hz, d); 7.17 (4H, J = 8.7 Hz, d); 6.89 (4H, J = 8.7 Hz, d); 6.07 (1H, J = 8.7 Hz, d); 3.72 (6H, s).

### 4C Preparation of bis(4-methoxyphenyl)methanamine hydrochloride (BMMA)

A reactor was charged with 2-propanol (2.5 L), afford *N*-(bis(4-methoxyphenyl)methyl)formamide (470 g, 1.73 mol), a 5 N solution of hydrochloric acid in 2-propanol (712 mL) and the contents of the reactor were refluxed for 1 h. In order to prevent the formation of a non stirable suspension, 2-propanol was added (4.5 L) and the reaction mixture was refluxed for 4 h. Heating was stopped and the reaction mixture was allowed to reach room temperature overnight under stirring. The temperature of the resulting suspension was set to 5 °C and the suspension was filtered. The wet cake was rinsed with 2-propanol (1.5 L) and was then dried under reduced pressure at 40 °C to afford bis(4-Methoxyphenyl)methanamine hydrochloride (400 g, 1.43 mol).
HPLC Method A_BEHC18: Ret. Time: 0.69 min; m/z 227 (pos)
¹H NMR (400 MHz, CDCl₃) δ (ppm): 7.27 (4H, J = 9.0 Hz, d); 6.83 (4H, J = 9.0 Hz, d); 4.99 (1H, s); 3.70 (6H, s).

### Example 5 Preparation of BMMA free base via BMMI

### 5A Preparation of bis(4-methoxyphenyl)methanimine (BMMI)

Tetrahydrofuran (120 mL) was added to 4-methoxybenzonitrile (77 g, 578 mmol) and the mixture was stirred until a clear solution is obtained. The clear solution was added over 15 min to a 1 M solution of 4-methoxyphenyl magnesium bromide in 2-methyltetrahydrofuran (700 mL, 700 mmol) at room temperature. The temperature of the resulting mixture was set to 40 °C to react for 3 h under stirring. The temperature was lowered to 5 °C and methanol (155 mL) was slowly added under stirring while keeping the temperature of the reaction below 30 °C. After completion of addition, water (175 mL) and aqueous 4 N hydrochloric acid were added in order to neutralize the pH of the reaction mixture. The mixture was then extracted twice with toluene (500 mL each). The combined organic layers were dried with brine (200 mL) and concentrated under reduced pressure to afford bis(4-methoxyphenyl)methanimine (138 g, 572 mmol).
HPLC Method A_BEHC18: Ret. Time: 0.71 min; m/z 242 (pos)
¹H NMR (400 MHz, CDCl₃) δ (ppm): 7.79 (4H, J = 8.8 Hz, d); 7.25 (4H, J = 8.8 Hz, d); 3.70 (6H, s).

### 5B Preparation of bis(4-methoxyphenyl)methanamine (BMMA free base)

bis(4-Methoxyphenyl)methanimine (138 g, 572 mmol) was dissolved in dry methanol (1 L) and sodium borohydride (11 g, 291 mmol) is added in three portions. After stirring for 3 h at room temperature reaction mixture was concentrated to a volume of about 200 mL. Ethyl acetate (250 mL) was slowly added to the concentrated residue and the organic layer was washed with water (100 mL). Aqueous 1 N hydrochloric acid (250 mL) was added to the organic layer and the resulting mixture was stirred for 15 min. The phases were allowed to settle and the aqueous phase was collected. This process was repeated once. The pH of the combined aqueous layers was adjusted to pH 12 and the aqueous layers were extracted with toluene (3 x 150 mL). The combined organic layers were dried with brine (150 mL) and concentrated under reduced pressure to afford bis(4-methoxyphenyl)methanamine (118 g, 485 mmol).

### Example 6 BMMA + DHP to yield the compound of Formula 1

A first reactor was charged with *N*-methyl-2-pyrrolidone (2 L) and with 4-hydroxy-2-(pyridazin-3-yl)pyrimidine-5-carboxylic acid (307 g, 1.41 mol). Triethylamine (392 mL, 2.81 mol) was added and the reaction mixture was stirred at ambient temperature until a clear solution is obtained. Following the addition of 1,1'-Carbonyldiimidazole (274 g, 1.69 mol), the temperature was set to 65 °C. In a second reactor *N*-methyl-2-pyrrolidone (770 mL) and bis(4-methoxyphenyl)methanamine hydrochloride (433 g, 1.55 mol) were stirred at ambient temperature until a solution was obtained. The solution of the second reactor was added to the solution of activated acid in the first reactor while maintaining the temperature to 65 °C. The second reactor was rinsed with *N*-methyl-2-pyrrolidone (500 mL) which was also added to the reaction mixture in the first reactor. The content of the first reactor was stirred at 65 °C for 1 h and ethanol (10 L) was added. The temperature was adjusted to 50 °C and aqueous 4 N hydrochloric acid (2.3 L) was added. Heating was stopped and the reaction mixture was allowed to reach room temperature and stirred for 15 h. The obtained suspension was filtered and the wet cake was rinsed with ethanol (4 L). The obtained solid was dried under reduced pressure at 40 °C to afford *N*-(bis(4-methoxyphenyl)methyl)-6-oxo-2-(pyridazin-3-yl)-1,6-dihydropyrimidine-5-carboxamide (622 g, 1.40 mol).

The quality of the isolated material was improved by applying the following process: A reactor was charged with 2-propanol (9 L), water (3 L) and *N*-(bis(4-methoxyphenyl)methyl)-6-oxo-2-(pyridazin-3-yl)-1,6-dihydropyrimidine-5-carboxamide (622 g, 1.40 mol). A 1 N aqueous sodium hydroxide solution was added in order to set the pH of the mixture to pH 13 and the temperature adjusted to 60 °C. A clear solution was obtained. The heating was stopped and 2 N Aqueous hydrochloric acid was added in order to adjust the pH of the mixture to below pH 2. The resulting suspension was filtered and the wet cake was sequentially rinsed with water (1 L) and with ethanol (1 L). The obtained solid was dried under reduced pressure at 40 °C to afford *N*-(bis(4-methoxyphenyl)methyl)-6-oxo-2-(pyridazin-3-yl)-1,6-dihydropyrimidine-5-carboxamide (560 g, 1.26 mol).HPLC Method A_BEHC18: Ret. Time: 0.95 min; m/z 442 (neg) ¹H NMR (400 MHz, CDCl₃) δ (ppm): 11.88 (1H, J = 8.6 Hz, d); 9.25 (1H, J = 1.7, 5.0 Hz, dd); 8.66 (1H, s); 3.70 (6H, s); 8.32 (1H, J = 1.7, 8.7 Hz, dd); 7.76 (1H, J = 5.0, 8.7 Hz, dd);.7.21 (4H, J = 8.6 Hz, d); 6.91 (4H, J = 8.6 Hz, d); 6.19 (1H, J = 8.3 Hz, d); 3.73 (6H, s).

## Claims

1. A process of making a compound of Formula I 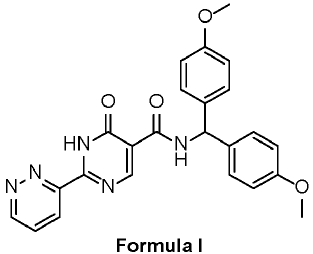 comprising
a) reducing a compound of Formula BMMI 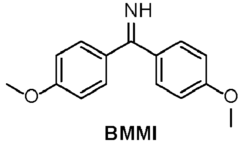 with a reducing agent to yield a compound of Formula BMMA free base 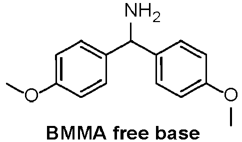
b) reacting the compound of Formula BMMA free base with a compound of Formula DHP 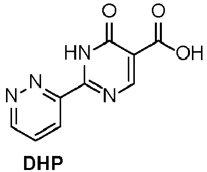 to yield the compound of Formula I.

2. A process of making a compound of Formula I 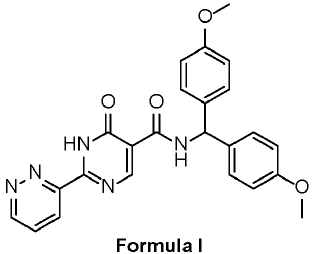 comprising
a) reacting pyridazine with between about 1.0 equivalent and about 1.5 equivalent of each of trimethylsilyl cyanide and toluenesulfonyl chloride to yield a compound of the Formula TPN 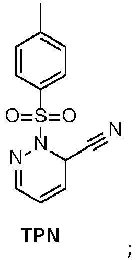
b) washing the compound of the Formula TPN with sodium bicarbonate to yield a compound of Formula PYN 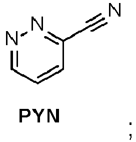
c) reacting the compound of Formula PYN with sodium in methanol at ambient temperature and then adding ammonium chloride to yield a compound of Formula PYA 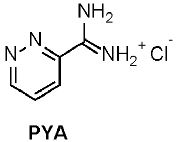
d) reacting the compound of Formula PYA with diethyl ethoxymethylenemalonate and sodium carbonate to yield a compound of Formula DHP 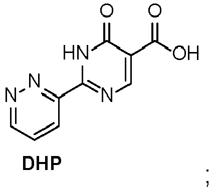 and
e) reacting the compound of Formula DHP with a compound of Formula BMMA 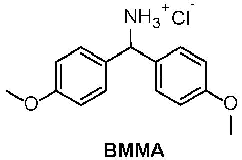 or a free base thereof to yield the compound of Formula I.

3. A process of making a compound of Formula BMMA 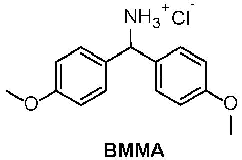 comprising
a) reacting a compound of Formula BMPN 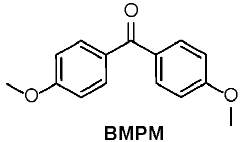 with formamide and formic acid to produce a compound of Formula BMPF 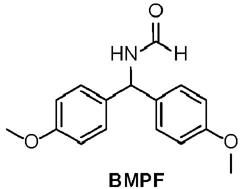 and
b) hydrolyzing the compound of Formula BMPF with an acid to yield the salt of the compound of Formula BMMA.

4. The process of claim 3, wherein the acid is selected from sulfuric acid, hydrobromic acid, phosphoric acid or hydrochloric acid.

5. A process of making a compound of Formula BMMA free base 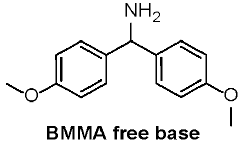 comprising
a) reducing a compound of Formula BMMI 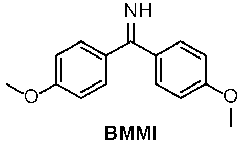 with a reducing agent to yield the compound of Formula BMMA free base.

6. The process of claim 5, wherein the reducing agent is sodium borohydride, a borane complexes, LiAlH₄) or catalytic hydrogenation with a Rhodium (Rh) or Iridium (Ir) catalyst.

7. A process of making a compound of Formula PYN 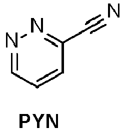 comprising
a) reacting pyridazine with between about 1.0 equivalent and about 1.5 equivalent of each of trimethylsilyl cyanide and toluenesulfonyl chloride to yield a compound of the Formula TPN 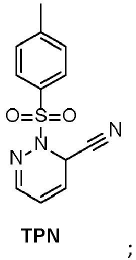 and
b) washing the compound of the Formula TPN with sodium bicarbonate to yield the compound of Formula PYN.

8. The process of claim 7, wherein the equivalents of each of trimethylsilyl cyanide and toluenesulfonyl chloride are about 1.2 equivalents.

9. The process of claim 7 or 8, wherein the process is conducted in a solvent of between about 8 to 15 volumes.

10. The process of claim 9, wherein the solvent is 2-methyltetrahydrofuran.

11. The process of anyone of claims 7 to 10, wherein the process of step a) further comprises adding aluminum trichloride in two, three or four portions.

12. A process of making a compound of Formula PYA 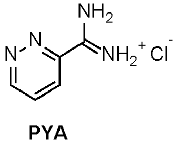 comprising
a) reacting a compound of Formula PYN with sodium in methanol at ambient temperature without reflux; and
b) adding ammonium chloride to yield the compound of Formula PYA.

13. A process of making a compound of Formula DHP 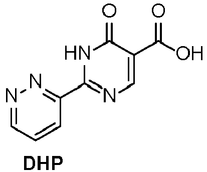 comprising reacting a compound of Formula PYA with diethyl ethoxymethylenemalonate and sodium carbonate to yield the compound of Formula DHP.

14. The process of claim 13, wherein the pH of the process is reduced to below about 3.5, preferably to 1.0.
